(19) Europäisches Patentamt
European Patent Office
Office européen des brevets

(11) **EP 4 717 281 A1**

(12) **EUROPÄISCHE PATENTANMELDUNG**

(43) Veröffentlichungstag:
**01.04.2026  Patentblatt 2026/14**

(21) Anmeldenummer: **24203666.3**

(22) Anmeldetag: **30.09.2024**

(51) Internationale Patentklassifikation (IPC):
*A61L 27/02* (2006.01)   *A61L 27/26* (2006.01)
*A61L 27/50* (2006.01)   *A61L 27/58* (2006.01)
*A61K 6/58* (2020.01)    *A61K 33/08* (2006.01)

(52) Gemeinsame Patentklassifikation (CPC):
(C-Sets verfügbar)
**A61L 27/025; A61K 33/08; A61L 27/26;**
**A61L 27/50; A61L 27/58;** A61L 2300/102;
A61L 2300/404; A61L 2400/06; A61L 2430/02
(Forts.)

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**GE KH MA MD TN**

(71) Anmelder: **Justus-Liebig-Universität Gießen,**
**Körperschaft des öffentlichen Rechts**
**35390 Gießen (DE)**

(72) Erfinder:
• **El Khassawna, Thaqif**
  **35398 Gießen (DE)**
• **Heiß, Christian**
  **35606 Solms (DE)**
• **Stötzel, Sabine**
  **35452 Heuchelheim (DE)**

(74) Vertreter: **Stumpf, Peter**
**c/o TransMIT GmbH**
**Kerkrader Strasse 3**
**35394 Gießen (DE)**

(54) **ANTIMIKROBIELLE ZUBEREITUNG**

(57)    Die Erfindung betrifft eine antimikrobielle, antiseptische und biologisch abbaubare Zubereitung, umfassend mindestens Calciumhydroxid, Polyvinylpyrrolidon (PVP) und Kollagen. Die Erfindung betrifft ebenfalls eine fließbare, injizierbare, antimikrobielle, antiseptische und biologisch abbaubare Paste, welche durch Zugabe von Wasser aus der Zubereitung hergestellt wird. Die Erfindung betrifft ebenfalls die Verwendung der Zubereitung und/oder der Paste als Medikament bei Gewebeinfektionen.

**EP 4 717 281 A1**

(52) Gemeinsame Patentklassifikation (CPC): (Forts.)

C-Sets
**A61L 27/26, C08L 39/06;**
**A61L 27/26, C08L 89/06**

## Beschreibung

Die vorliegende Erfindung befasst sich mit der Entwicklung eines neuen Biomaterials, das antimikrobielle Eigenschaften hat, biologisch abbaubar ist und in der Medizin zur Unterstützung von Heilungsprozessen z.B. zur Wundheilung, zur Knochenheilung und bei der Einpflanzung eines Implantates in den Körper eingesetzt werden kann. Ebenso soll die erfindungsgemäße Zubereitung den Knochenverlust in Folge einer Periimplantitis (implantatassoziierte Infektion) verhindern oder rückgängig machen und den Knochenaufbau nach der Einpflanzung eines Implantats fördern und damit die Einheilung des Implantats in den Knochen beschleunigen.

Implantate (=Endoprothesen) sind künstliche Materialien, die permanent oder für längere Zeit in einen menschlichen oder tierischen Körper eingepflanzt werden, um dort sensorische, neurologische, kardiovaskuläre, orthopädische, schmerzstillende, kontrazeptive oder kosmetische Funktionen zu übernehmen. Beispiele dafür sind verschiedene Formen des Gelenksersatzes (z.B. Hüftgelenksendoprothesen), Implantate für eine Osteosynthese (z.B. Platten und Schrauben) und Zahnimplantate als Befestigungsanker für künstliche Zähne. Eine potentielle Nebenwirkung bei der Implantation von Endoprothesen ist die Kontamination des Implantats oder des Operationsfeldes mit Bakterien oder anderen Krankheitserregern, welche zu einer Periimplantitis oder zu Knochen-Implantat-Infektionen führen. Unfallchirurgen, Orthopäden und Kieferchirurgen sind häufig mit solchen implantatassoziierten (implantatbedingten) bakteriellen Infektionen (Periimplantitis) konfrontiert, insbesondere bei Implantaten im Knochenbereich. Daher besteht ein großer Bedarf an einem Material zur Verhinderung oder Behandlung von Gewebeinfektionen in Folge der Implantation einer Endoprothese.

Die Zahl schwerer bakterieller Infektionen nimmt seit Jahren mit der Zahl der chirurgischen Eingriffe zu. Das Auftreten einer Periimplantitis bei einem Patienten hat schwerwiegende klinische Folgen, die zu Heilungsverzögerungen, Amputationen und sogar zum sepsisbedingten Tod führen können. Wenn eine Periimplantitis auftritt, muss häufig das infizierte Implantat wieder chirurgisch entfernt werden. Das verursacht nicht nur eine zusätzliche Belastung für den betroffenen Patienten, sondern erhöht auch die gesundheitsökonomischen Kosten.

Eine schwerwiegende Auswirkung einer Periimplantitis ist der Knochenverlust durch die Infektion. Dieser Knochenverlust hat schwerwiegende Folgen für den betroffenen Patienten und steht daher im besonderen Fokus der Implantat-Chirurgie.

## Stand der Technik

Um Periimplantitiden zu verhindern oder die Folgen einer Periimplantitis (wie z.B. Knochenverlust) zu behandeln, müssen die Art des Implantats und seine Materialeigenschaften beachtet werden. Beispielsweise können durch eine bioaktive Beschichtung, eine Oberflächenmodifikation oder eine Nano-Silber-Technologie Implantate so verändert werden, dass die Gefahr einer Periimplantitis verringert wird. Die Beschichtungen bzw. Modifikationen der Implantate erhöhen jedoch die Kosten der Implantate, und es liegen nur wenige klinische Daten zur Effektivität dieser Maßnahmen vor.

Der Einsatz lokaler Antibiotika bei der Vorbeugung und Behandlung von Periimplantitiden gilt als wirksam und hat gegenüber der systemischen Antibiotikatherapie den Vorteil, dass die Kosten niedriger sind und an den gewünschten Stellen wesentlich höhere Konzentrationen des Antibiotikums erreicht werden. In der Regel muss jedoch das infizierte Implantat vor der lokalen Anwendung eines Antibiotikums chirurgisch entfernt werden. Bei den meisten derzeitigen Behandlungsmethoden ist anschließend ein zweiter Eingriff zur Entfernung des Antibiotika-Trägers erforderlich, so dass insgesamt vier zusätzliche chirurgische Eingriffe erforderlich sind (Entfernung des Implantats, lokale Behandlung, Entfernung des Trägers und schließlich ein neues Implantat). Zusätzlich sind Antibiotika-Allergien bekannt und schränken die Nutzung von Antibiotika weiter ein. Außerdem treten vermehrt Resistenzen von Bakterien gegen bestimmte Antibiotika auf.

Obwohl die chirurgische Therapie einer Periimplantitis, nämlich die Implantatentfernung und Neuimplantation nach Ausheilung der Infektion als die wirksamste Therapieoption beschrieben wird, liegt die Erfolgsrate nur bei ca. 50%. Die dadurch entstehenden Kosten müssen die Patienten selber tragen, oder werden von den Krankenkassen übernommen. Beispielsweise belaufen sich die durchschnittlichen Kosten für ein einzelnes Zahnimplantat auf etwa 2.500€ im Falle einer Periimplantitis und einer chirurgischen Implantatentfernung. Darüberhinaus muss der Patient 6-12 Monate auf die Ausheilung der Periimplantitis warten, bevor ein neues Zahnimplantat eingesetzt werden kann. Danach ist noch einmal eine 4-6 Monate lange Wartezeit nötig, bis der prothetische Teil des Zahnersatzes eingesetzt werden kann. Die Gesamtkosten belaufen sich hier auf über 5.000€ für das verlorene und das neue Zahnimplantat. Zusätzlich muss sich der Patient mehreren Operationen unterziehen und die Wartezeit bis zum Einsatz des Zahnimplantates verlängert sich sehr stark.

Ein anderer Ansatz ist die Verwendung von Medizinprodukten, welche einer Periimplantitis durch Antisepsis vorbeugen sollen. Einige dieser nicht antibiotischen Medizinprodukte sind kommerziell erhältlich, z.B. Calciumhydroxid und Jodoform (laut IUPAC-Nomenklatur Iodoform). Jodoform kann auch als Gel zur Verfügung gestellt werden. Sie werden jedoch meist zur Reinigung von Defektbereichen verwendet, ohne dass zuverlässige klinische Daten zu ihrer langfristigen Wirkung gegen Periimplantitis vorliegen. Calciumhydroxid ist von Natur aus stark alkalisch und kann dementsprechend Bakterien abtöten. Es hat eine entzündungshemmende und sogar antimikrobielle Wirkung. Aufgrund der Wirkung von Calciumhydroxid wird es in der Zahnmedizin bei der Wurzelkanalbehandlung eingesetzt. Die Erfolgsquote bei der Behandlung von Entzündungen während einer Wurzelkanalbehandlung wird mit 96% angegeben. Dementsprechend wird Calciumhydroxid klinisch zur Behandlung periapikaler Läsionen eingesetzt, um die Heilung zu fördern. Calciumhydroxid weist allerdings bei der Verwendung als antimikrobielle Zubereitung folgende Nachteile auf:

- Zersetzung in Gegenwart von Jodoform: Calciumhydroxid kann Jodoform in stark basischer Umgebung zersetzen, was die bakterizide Wirkung von Jodoform verringert. Die Freisetzung von hochreaktiven Zwischenprodukten wie Dijodcarben führt zu einer verminderten Stabilität der Zubereitung.
- Toxische Eigenschaften: Calciumhydroxid hat starke basische Eigenschaften, die bei höheren Konzentrationen zu Gewebeschäden und toxischen Reaktionen führen können, was die Heilung beeinträchtigt und unerwünschte Nebenwirkungen hervorruft.
- Fehlende Bioabbaubarkeit: Calciumhydroxid ist nicht vollständig biologisch abbaubar, was bedeutet, dass es im Körper verbleiben und langfristige Reaktionen oder Komplikationen verursachen kann.
- Unzureichende Unterstützung der Knochenregeneration: Allein verwendet, bietet Calciumhydroxid keine ausreichende Unterstützung für die Knochenregeneration. Es fehlen die notwendigen biologischen Prozesse zur Förderung der Heilung und Integration von Implantaten.
- Mangelnde Viskosität und Fließeigenschaften: Calciumhydroxid hat nicht die gewünschten Viskositäts- und Fließeigenschaften für eine gleichmäßige Verteilung, was zu einer schnellen Aushärtung oder ungleichmäßigen Verteilung führen kann. Damit ist die Wirksamkeit einer Zubereitung mit Calciumhydroxid unzuverlässig.

Jodoform wird in Kontakt mit Gewebe allmählich abgebaut, wobei freies Jod in die Gewebsflüssigkeit freigesetzt wird. Es hat dadurch eine stark desinfizierende Wirkung, analog wie etwa Jod/Jodkaliumlösung, die direkt freies Jod enthält. Es ist gleichermaßen gut geeignet, um Infektionen vorzubeugen und die Heilung zu fördern. Daher wird dieses Antiseptikum in der zahnärztlichen Praxis bei zystischen Defekten und zur Heilung von Weichgewebe eingesetzt. Das Jod wirkt schmerzlindernd und kann leicht absorbiert werden. Jodoform weist allerdings bei der Verwendung als antimikrobielle Zubereitung folgende Nachteile auf:

- Unkontrollierte Freisetzung von Jod: Jodoform wird in Kontakt mit Gewebe allmählich abgebaut, wobei freies Jod in die Gewebsflüssigkeit freigesetzt wird. Dies kann zu einer starken desinfizierenden Wirkung führen, aber auch zu einer unkontrollierten und möglicherweise schädlichen Freisetzung von Jod, was die Sicherheit und Wirksamkeit der Anwendung beeinträchtigen kann.
- Fehlende Langzeitdaten: Es fehlen zuverlässige klinische Daten zur langfristigen Wirkung von Jodoform gegen Periimplantitis. Ohne diese Daten ist es schwierig, die Langzeitwirksamkeit und -sicherheit von Jodoform als alleinige Behandlung zu beurteilen.

- Begrenzte antibakterielle Wirkung: Während Jodoform eine desinfizierende Wirkung hat, ist es nicht so wirksam wie andere kombinierte antimikrobielle Präparate. Seine alleinige Anwendung ist daher nicht ausreichend, um eine vollständige Beseitigung von Bakterien zu gewährleisten, die für eine erfolgreiche Behandlung von Infektionen erforderlich ist.
- Keine knochenregenerierende Wirkung: Jodoform hat keine knochenaufbauende Wirkung. Daher ist es allein nicht geeignet, um den Knochenaufbau nach der Einpflanzung eines Implantats zu fördern oder den Knochenverlust in Folge einer Periimplantitis zu verhindern oder rückgängig zu machen.
- Toxische Eigenschaften: Jodoform kann in höheren Konzentrationen toxisch sein und zu Gewebeschäden führen. Dies kann die Heilung beeinträchtigen und zu unerwünschten Nebenwirkungen führen, insbesondere bei langfristiger Anwendung.

Kollagen (Collagen) ist ein Eiweiß, das sich als Strukturprotein zu faserförmigen Bündeln anordnet und die Grundlage des Bindegewebes bzw. der extrazellulären Matrix bei Tieren und Menschen bildet. Es sind verschiedene Arten von Kollagenen bekannt. Kollagene finden sich unter anderem in den weißen, unelastischen Fasern von Sehnen, Bändern, Knochen, Knorpeln und auch in der Unterhaut. Kollagene können im Körper von Tieren und Menschen enzymatisch abgebaut werden, beispielsweise durch Kollagenasen aus Fibrozyten. Kollagene werden beispielsweise in der chemischen und pharmazeutischen Industrie für die Umhüllungen von Tabletten (Hart- und Weichkapseln) sowie für Gelatinezäpfchen verwendet. Außerdem wird Kollagen in Form von Gelatine für blutstillende Schwämmchen sowie als Blutplasma-Ersatz eingesetzt. Kollagen weist allerdings bei der Verwendung als antimikrobielle Zubereitung folgende Nachteile auf:

- Mangelnde antimikrobielle Wirkung: Kollagen hat keine ausreichende antimikrobielle Wirkung, um Gewebeinfektionen zu verhindern oder zu behandeln. Dies bedeutet, dass es allein nicht wirksam gegen bakterielle Infektionen ist, die bei Implantationen auftreten können.
- Fehlende Langzeitstabilität: Kollagen kann im Körper enzymatisch abgebaut werden, was seine langfristige Stabilität und Wirksamkeit beeinträchtigen kann. Diese Eigenschaft macht es ungeeignet für Anwendungen, die eine dauerhafte Präsenz im Gewebe erfordern.
- Begrenzte mechanische Eigenschaften: Kollagen allein hat nicht die erforderlichen mechanischen Eigenschaften, um als stabiles Trägermaterial in medizinischen Anwendungen zu dienen. Es kann unter Belastung schnell seine Form verlieren oder abgebaut werden.
- Fehlende knochenregenerierende Wirkung: Kollagen allein hat keine ausreichende Fähigkeit, die Knochenregeneration zu fördern. Es muss mit anderen Substanzen kombiniert werden, die diese Eigenschaft unterstützen, um bei der Heilung und Regeneration von Knochen wirksam zu sein.

Polyvinylpyrrolidon (PVP), auch Povidon oder Polyvidon genannt, ist ein lineares Polymer der Verbindung Vinylpyrrolidon. Vinylpyrrolidon weist die Summenformel $C_6H_9NO$ auf. Die CAS-Nummer von Polyvinylpyrrolidon lautet 9003-39-8. PVP ist ein hygroskopisches, amorphes Pulver mit weißer bis hellgelber Farbe. Die Molmassen der handelsüblichen Polymere liegen im Bereich von ca. 2.500 bis 2.500.000 Dalton. Polyvinylpyrrolidon wird als Hilfsstoff in der pharmazeutischen Industrie eingesetzt, beispielsweise als Bindemittel für Tabletten, in Augentropfen oder als Blutplasmaexpander. Polyvinylpyrrolidon (PVP) weist allerdings bei der Verwendung als antimikrobielle Zubereitung folgende Nachteile auf:

- Mangelnde antimikrobielle Wirkung: PVP alleine hat keine ausreichende antimikrobielle Wirkung, um Infektionen zu verhindern oder zu behandeln. Es bietet keinen Schutz gegen bakterielle Infektionen, was seine alleinige Anwendung in medizinischen Präparaten einschränkt.
- Keine knochenregenerierende Wirkung: PVP allein hat keine Fähigkeit, die Knochenregeneration zu fördern. Es unterstützt nicht den Aufbau und die Heilung von Knochengewebe, was bei der Behandlung von Knochenerkrankungen oder Implantationen notwendig ist.
- Begrenzte mechanische Eigenschaften: PVP allein bietet nicht die erforderlichen mechanischen Eigenschaften, um als stabiles Trägermaterial in medizinischen Anwendungen zu dienen. Es kann nicht die gewünschte Festigkeit und Stabilität aufweisen.
- Fehlende Bioabbaubarkeit: Obwohl PVP in einigen Anwendungen als biologisch verträglich gilt, ist es nicht vollständig biologisch abbaubar, was zu langfristigen Rückständen im Körper führen kann. Dies kann mögliche Komplikationen und negative Langzeiteffekte verursachen.

Eine Mischung aus Polyvinylpyrrolidon mit 3% Jod wird in Form einer Lösung, Salbe oder Creme in der Wundversorgung verwendet, beispielsweise unter den Handelsnahmen Betaisodona® oder Braunol®. Diese Zubereitungen haben allerdings keine knochenaufbauende Wirkung und weisen auch die Nachteile des Polyvinylpyrrolidons auf.

EP 3 273 948 B1 beschreibt eine Zubereitung, die antimikrobielle Eigenschaften hat. Die Applikation erfolgt in Kapselform. Allerdings ist die Zubereitung nicht biologisch abbaubar und nicht zur Behandlung von Knochen vorgesehen.

EP 2 683 421 B1 beschreibt eine antibiotikahaltige Implantatbeschichtung, die allerdings nicht zur Anwendung am Knochen eines Patienten geeignet ist. Außerdem handelt es sich bei einer Oberflächenbeschichtung nicht um eine pharmazeutische Zubereitung.

EP 1 244 434 B1 offenbart Chemotherapeutika für die Behandlung von Wunden im Dentalbereich und stellt damit ebenfalls eine Zubereitung zur Verfügung, die antimikrobielle Eigenschaften hat. Die Applikation erfolgt zwar wahlweise in Gelform, ist aber nicht biologisch abbaubar und nicht zur Behandlung von Knochen vorgesehen.

**Aufgaben**

Das Ziel dieser Erfindung ist es, eine injizierbare, antiseptische, antimikrobielle und biologisch abbaubare Zubereitung zur Verfügung zu stellen, die medizinisch zur Verhinderung oder zur Behandlung von Periimplantitiden (implantatassoziierten Infektionen), insbesondere im Knochenbereich, eingesetzt werden kann. Ebenso soll die erfindungsgemäße Zubereitung den Knochenverlust in Folge einer Periimplantitis verhindern oder rückgängig machen. Ebenso soll die erfindungsgemäße Zubereitung den Knochenaufbau nach der Einpflanzung eines Implantats fördern und damit die Einheilung des Implantats in den Knochen beschleunigen, also die Knochenregenerationsfähigkeit stimulieren. Insgesamt sollen mit der Erfindung sowohl die Häufigkeit als auch die Schwere von Periimplantitiden verringert werden, deren Folgen reduziert oder rückgängig gemacht werden und die Einheilung eines Implantates in den Knochen verbessert werden.

**Lösung der Aufgaben**

Diese Aufgaben werden erfindungsgemäß gelöst durch eine Zubereitung gemäß den Ansprüchen. Die Zubereitung umfasst Calciumhydroxid, Polyvinylpyrrolidon (PVP) und Kollagen. Die erfindungsgemäße Zubereitung umfasst folgende Gewichtsprozente: 30,0-40,0% w/w Calciumhydroxid, 17,0-22,5% w/w Polyvinylpyrrolidon und 27,0-37,5% w/w Kollagen. In einer ersten Ausführungsform liegt die erfindungsgemäße Zubereitung in Pulverform vor. In einer Ausführungsform kann die erfindungsgemäße Zubereitung mit Wasser zu einer fließfähigen Paste verarbeitet werden. In einer weiteren Ausführungsform handelt es sich um destilliertes Wasser. In einer weiteren Ausführungsform handelt es sich um steriles Wasser. In einer weiteren bevorzugten Ausführungsform handelt es sich um destilliertes steriles Wasser. Unter Berücksichtigung des beigefügten Wassers betragen die Gewichtsprozente der aus der erfindungsgemäßen Zubereitung hergestellten erfindungsgemä-βen Paste 30,0-34,0% w/w Calciumhydroxid, 17,0-19,0% w/w Polyvinylpyrrolidon, 27,0-31,0% w/w Kollagen und 16,5-18,0% w/w Wasser.

Die Kombination von Calciumhydroxid und Kollagen in der Zubereitung gewährleistet eine effektive Abtötung und Hemmung des Wachstums von Mikroorganismen. Während Calciumhydroxid durch seine alkalische Wirkung die Mikroorganismen zerstört, unterstützt Kollagen die antimikrobielle Wirkung durch physikalische Hemmung und potenziell durch spezifische antimikrobielle Peptide. Polyvinylpyrrolidon trägt zur Stabilisierung und besseren Verteilung der Wirkstoffe bei, was die Wirksamkeit der Zubereitung weiter verbessert.

Die erfindungsgemäße Zubereitung und die aus der erfindungsgemäßen Zubereitung hergestellte erfindungsgemäße Paste sind biologisch abbaubar. Die biologische Abbaubarkeit wird hauptsächlich durch Kollagen verursacht. Polyvinylpyrrolidon (PVP) trägt ebenfalls zur biologischen Abbaubarkeit bei.

Die erfindungsgemäße Zubereitung ist antiseptisch und antimikrobiell. Die aus der erfindungsgemäßen Zubereitung hergestellte erfindungsgemäße Paste ist injizierbar, antiseptisch und antimikrobiell.

Die erfindungsgemäße Zubereitung und die aus der erfindungsgemäßen Zubereitung hergestellte erfindungsgemäße Paste sind geeignet zur Verhinderung oder zur Behandlung von Periimplantitiden (implantatassoziierten Infektionen), insbesondere im Knochenbereich.

Die erfindungsgemäße Zubereitung und die aus der erfindungsgemäßen Zubereitung hergestellte erfindungsgemäße Paste verhindern den Knochenverlust in Folge einer Periimplantitis oder machen den Knochenverlust in Folge einer Periimplantitis rückgängig.

Die erfindungsgemäße Zubereitung und die aus der erfindungsgemäßen Zubereitung hergestellte erfindungsgemäße Paste fördern den Knochenaufbau nach der Einpflanzung eines Implantats und beschleunigen damit die Einheilung des Implantats in den Knochen, stimulieren also die Knochenregenerationsfähigkeit.

Die erfindungsgemäße Zubereitung und die aus der erfindungsgemäßen Zubereitung hergestellte erfindungsgemäße Paste verringern sowohl die Häufigkeit als auch die Schwere von Periimplantitiden, reduzieren deren Folgen oder machen diese rückgängig und verbessern die Einheilung eines Implantates in den Knochen. Die erfindungsgemäße Zubereitung und die aus der erfindungsgemäßen Zubereitung hergestellte erfindungsgemäße Paste bewirken eine gute Knochenregenerationsfähigkeit. Unter Knochenregeneration wird das Wachstum neuer Knochenzellen und die Bildung von neuem Knochengewebe verstanden. Die Knochenregeneration ist von vielen Faktoren abhängig, welche dem Fachmann bekannt sind und deren qualitative Beurteilung ebenso dem Fachmann bekannt ist.

Die Kombination von Kollagen und Calciumhydroxid in der erfindungsgemäßen Zubereitung gewährleistet die Knochenregenerationsfähigkeit. Kollagen bietet die strukturelle Unterstützung für das Wachstum neuer Knochenzellen und Gewebe, während Calciumhydroxid die Mineralisierung und die Bildung von neuem Knochengewebe durch die Bereitstellung von Kalziumionen fördert und eine förderliche Umgebung für die Knochenheilung schafft.

Die erfindungsgemäße Zubereitung und die aus der erfindungsgemäßen Zubereitung hergestellte erfindungsgemäße Paste sind geeignet, prophylaktisch oder therapeutisch an Menschen oder Wirbeltieren angewendet zu werden. Sie lösen damit alle an sie gestellten Aufgaben.

Im Rahmen eigener wissenschaftlicher Forschung ist es gelungen, eine Zubereitung bereitzustellen, welche die toxischen Eigenschaften von Calciumhydroxid reduziert. Dies erfolgte durch Kombination mit Kollagen, wodurch eine hohe antimikrobielle Wirkung, kombiniert mit einer sehr guten biologischen Abbaubarkeit und Stimulierung der Knochenregenerationsfähigkeit erreicht wird.

Es wurde gefunden, dass Kollagen, Calciumhydroxid und Polyvinylpyrrolidon mit Wasser zu einer fließfähigen Paste vermischt werden können, die verschiedene vorteilhafte medizinische Eigenschaften aufweist. Ohne an eine konkrete Theorie gebunden zu sein, kann vermutet werden, dass diese spezielle Kombination von Calciumhydroxid mit Kollagen und Polyvinylpyrrolidon zu einer unerwarteten Stabilisierung der einzelnen Komponenten führt, die so nicht erwartet werden konnte. Diese Stabilisierung kann vermutlich auf die Wirkung von Kollagen und Polyvinylpyrrolidon in Kombination miteinander als eine Art "Schutzkolloid" zurückgeführt werden. Dadurch ist die erfindungsgemäße Zubereitung nahezu unbegrenzt haltbar. Die aus der erfindungsgemäßen Zubereitung hergestellte erfindungsgemäße Paste hat eine Haltbarkeit von mindestens 3 Wochen.

Eine Wirkung von polymeren Substanzen als Schutzkolloide ist zwar in anderen Bereichen bereits bekannt, konnte hier aber nicht a priori erwartet werden, da die stark basische Wirkung des Calciumhydroxids erwartungsgemäß zersetzende Wirkung auch auf Kollagen als Eiweiß haben und somit zu seiner raschen Zerstörung führen sollte. Die Bindung des Wasseranteils in der erfindungsgemäßen Paste durch die Bildung eines Schutzkolloids hat den technischen Effekt, dass das Calciumhydroxid stabilisiert wird, denn die im Schutzkolloid gebundenen Wassermoleküle stehen nicht mehr für eine Umsetzung des aus der Umgebung stammenden Kohlendioxids mit dem Calciumhydroxid zur Verfügung, so dass dessen Umsetzung zu Calciumcarbonat verzögert wird.

Die unerwartete Stabilität und Wirksamkeit der aus der erfindungsgemäßen Zubereitung hergestellte erfindungsgemäße Paste kann - zumindest teilweise - auf den pastösen Charakter zurückgeführt werden, da das Kollagen und das Polyvinylpyrrolidon die in der pastösen Zubereitung enthaltenen Wassermoleküle durch Ausbildung von Wasserstoffbrückenbindungen binden.

**Herstellung der aus der erfindungsgemäßen Zubereitung hergestellte erfindungsgemäße Paste:**

Die erfindungsgemäße Zubereitung besteht aus einem Gemisch aus Calciumhydroxid, Polyvinylpyrrolidon (PVP) und Kollagen. Aus dieser Zubereitung wird durch Anmischen mit Wasser, beispielsweise mit destilliertem und/oder sterilem Wasser, eine fließfähige Paste hergestellt. Dazu wurde in einem bevorzugten Ausführungsbeispiel eine Paste mit einem Verhältnis von 40,0% w/w Calciumhydroxid, 22,5% w/w Polyvinylpyrrolidon und 37,5% w/w Kollagen angemischt. Um die Paste herzustellen, wurden die Bestandteile Calciumhydroxid, Polyvinylpyrrolidon-Pulver und Kollagenpulver in den angegebenen Mengenverhältnissen abgewogen und mit einer ausreichenden Menge Wasser vermischt, so dass eine Paste entsteht.

Für die erfindungsgemäße Zubereitung hat sich das Polyvinylpyrrolidon PVP10, also eines Polyvinylpyrrolidons mit einer Molekularmasse von ca. 10.000 Dalton, als besonders gut geeignet erwiesen. Im Rahmen der durchgeführten Versuche zeigte sich, dass die Verwendung von PVP10 die Fließeigenschaften, das Abbindeverhalten und die Haltbarkeit der Zubereitung positiv beeinflusst. Zusätzlich hat PVP10 noch folgende Vorteile gegenüber anderen PVPs:

- Niedrigeres Molekulargewicht: PVP10 hat ein niedrigeres Molekulargewicht (ca. 10.000 Dalton) im Vergleich zu anderen PVP-Varianten wie PVP40. Dies führt zu besseren Fließeigenschaften und einer niedrigeren Viskosität der Paste, was die Handhabung und Anwendung erleichtert.
- Bessere Löslichkeit: PVP10 ist in Wasser besser löslich als Varianten mit höherem Molekulargewicht. Dies führt zu einer gleichmäßigeren Verteilung in der Paste und gewährleistet eine konsistente und homogene Mischung der Inhaltsstoffe.
- Bessere Stabilität: PVP10 trägt zur Stabilisierung der anderen Komponenten der Paste bei, insbesondere bei der Kombination mit Kollagen und Calciumhydroxid. Diese Stabilität ist wichtig, um die Haltbarkeit und Wirksamkeit der Paste über längere Zeiträume zu gewährleisten.
- Reduzierte Viskosität: Die niedrigere Viskosität von PVP10 ermöglicht eine einfachere Injektion der Paste, was besonders wichtig für medizinische Anwendungen ist, bei denen eine präzise und kontrollierte Applikation erforderlich ist.
- Biokompatibilität: PVP10 ist biokompatibel und wird in vielen pharmazeutischen und medizinischen Anwendungen verwendet, da es gut verträglich ist und keine unerwünschten Reaktionen im Körper hervorruft.
- Feuchtigkeitsbindung: PVP10 hat hervorragende feuchtigkeitsbindende Eigenschaften, was die Hydratation und Stabilität der Paste verbessert und dazu beiträgt, die gewünschten physikalischen Eigenschaften der Paste zu erhalten.

Das Molekulargewicht des verwendeten Polyvinylpyrrolidons hat direkt Einfluss auf die Fließeigenschaften (Viskosität, Fließwiderstand) der pastösen Zubereitung. Falls andere Fließeigenschaften gewünscht sind, oder eine andere Variante von Kollagen verwendet werden soll, kann aber auch eine andere Variante von Polyvinylpyrrolidon eingesetzt werden (etwa PVP40), um die gewünschten Fließeigenschaften zu erreichen. Fachkundigen Personen ist bekannt, dass die Fließeigenschaften pastöser Zubereitungen (Pasten) von den Eigenschaften und Mengenverhältnisse der Ausgangsstoffe abhängig sind, so dass es für die Einstellung der Fließeigenschaften immer mehrere Variationsmöglichkeiten hinsichtlich der Varianten und Anteile der Ausgangsstoffe gibt. Auch die gemeinsame Verwendung von verschiedenen PVPs wie beispielsweise PVP10 und PVP40 ist möglich.

Folgende Zubereitungen sind möglich, um bestimmte gewünschte Eigenschaften zu erzielen:

- Kombination von PVP10 und PVP40: Diese Kombination hat den Vorteil, dass sie eine ausgewogene Viskosität und bessere mechanische Eigenschaften bietet. PVP10 sorgt für eine gute Löslichkeit und einfache Verarbeitung, während PVP40 die Festigkeit und Stabilität der Paste erhöht. Diese Kombination kann verwendet werden, wenn sowohl eine gute Injektionsfähigkeit als auch eine erhöhte mechanische Festigkeit der Paste erforderlich sind.
- Kombination von PVP10 und PVP K30: Diese Kombination hat den Vorteil, dass PVP K30 ein mittleres Molekulargewicht hat (ca. 30.000 Dalton) und kann daher ein Kompromiss zwischen der Verwendung von PVP10 und PVP40 sein. Es kombiniert die gute Löslichkeit von PVP10 mit einer moderaten Erhöhung der Viskosität und Festigkeit. Diese Kombination ist nützlich, wenn eine Paste mit mittlerer Viskosität und ausreichender Stabilität benötigt wird.

- Verwendung von PVP VA Copolymeren: Diese Copolymere von PVP und Vinylacetat (z.B. PVP/VA S630) können spezifische Eigenschaften wie verbesserte Adhäsion und Flexibilität bieten. Diese Copolymere sind besonders vorteilhaft in Anwendungen, bei denen eine gute Haftung auf Gewebe erforderlich ist. Sie sind ideal für Situationen, in denen die Paste fest an Ort und Stelle bleiben muss, wie bei der Behandlung von Knochendefekten oder für die Wundheilung.

- Kombination von PVP10 und PVP K90: PVP K90 hat ein hohes Molekulargewicht (ca. 90.000 Dalton) und kann die Viskosität und die mechanische Festigkeit erheblich erhöhen. In Kombination mit PVP10 kann eine kontrollierte Anpassung der Fließeigenschaften erreicht werden. Diese Kombination kann verwendet werden, wenn eine sehr feste und stabile Paste benötigt wird, die dennoch ausreichend fließfähig ist, um injiziert zu werden.

Durch die Anpassung der Mengenverhältnisse von Kollagen, Calciumhydroxid und PVPs kann die Fließeigenschaften weiter optimiert werden. Zum Beispiel kann ein höherer Anteil an PVP10 die Löslichkeit und Injektionsfähigkeit verbessern, während ein höherer Anteil an PVP40 oder PVP K90 die Festigkeit erhöht.

Durch die Einführung von Zusatzstoffen kann ebenfalls die Fließeigenschaften angepasst werden, um spezifische Anforderungen zu erfüllen. Dies kann beispielsweise durch die Zugabe von plastifizierenden oder verdickenden Agenten wie Glycerin oder Polyethylenglykol (PEG) erreicht werden.

**Herstellung im Labormaßstab:**

Bei allen nachfolgend beispielhaft genannten Prozentangaben handelt es sich um Angaben in Gewichtsprozent (% w/w), auch wenn es sich um Flüssigkeiten handelt. Um die erfindungsgemäße pastöse Zubereitung im Labormaßstab herzustellen, werden 3,37 g Kollagenpulver, 4 g Calciumhydroxid sowie 2,25 g Polyvinylpyrrolidon-Pulver abgewogen. Damit eine fließfähige, aber feste Masse entsteht (fest im Sinne von pastös, d.h. formbar und zumindest teilweise gestalthaltend für wenigstens wenige Sekunden), wird jede Substanz mit so wenig destilliertem und sterilen Wasser versetzt und vermengt, dass eine fließfähige aber trotzdem formbare und gestalterhaltende Paste entsteht. Diese Paste ist mindestens für einige Stunden fließfähige aber trotzdem formbar und gestalterhaltend. Die Paste weist also thixotrope Eigenschaften auf, weil sie unter Scherung fließfähiger wird und im Ruhezustand wieder eine festere Form annimmt. Dieses thixotrope Verhalten ist besonders vorteilhaft, weil sie dadurch gut für einen 3D-Druck geeignet ist.

Tabelle 1: Trockene Substanzen der erfindungsgemäßen Zubereitung zur Herstellung einer Paste sowie beispielhafte Werte der erforderlichen Wasserzugabe (in ml pro Gramm der jeweiligen Substanz), die - je nach Feuchtigkeitsgehalt und/oder Körnungsgrad der Ausgangssubstanzen - um bis zu 20% um den jeweiligen angegebenen Wert schwanken können.

| Substanz [pro Gramm] | Destilliertes steriles Wasser [ml] |
|---|---|
| Kollagen | 0,7 |
| Calciumhydroxid | 1,0 |
| Polyvinylpyrrolidon | 0,3 |

Das Calciumhydroxidpulver wird auf einer sauberen Unterlage ausgelegt und mit einer Einweg-Pipette tröpfchenweise mit abgemessenem destilliertem sterilem Wasser befeuchtet. Mit Hilfe eines Spatels wird das Calciumhydroxidpulver mit dem Wasser vermengt. Schrittweise wird Wasser zu dem Calciumhydroxid dazugegeben, sodass eine pastöse, mischbare Masse gebildet wird. Darauffolgend wird das Kollagen tropfenweise mit abgemessenem destilliertem sterilem Wasser versetzt und mit einem Spatel vermengt. Es wird nur so viel Wasser hinzugegeben, bis sich eine Paste gebildet hat. Diese Paste soll eine fließfähige, aber formbare Konsistenz haben, die als "pastös" und "krümelig klebend" beschrieben werden kann. Diese Konsistenz ermöglicht es der Paste, ihre Form für einige Sekunden zu halten, bevor sie sich unter Scherung verformt und bei Bedarf wieder ihre ursprüngliche Form annimmt.

Anschließend wird Polyvinylpyrrolidon-Pulver in kleinen Mengen mit abgemessenem destilliertem sterilem Wasser angereichert, um eine pastöse Masse zu bilden. Danach werden alle Substanzen zusammengeführt und vermengt. Das Vermengen wird so lange durchgeführt, bis das Kollagen, das Calciumhydroxid und das Polyvinylpyrrolidon eine feste, aber noch fließfähige (d.h. pastöse) Masse gebildet haben. Zu der vermengten Menge werden weitere 0,1 ml destilliertes steriles Wasser dazugegeben, damit die pastöse Zubereitung fließfähig ist. Daraufhin wird die pastöse Zubereitung in ein geeignetes Gefäß, z.B. in Einmalspritzen gefüllt und ist gebrauchsfertig.

Die Viskosität und der Fließwiderstand der erfindungsgemäßen Zubereitung, also der Paste, lässt sich durch folgende allgemeine Formel verdeutlichen:

$$F = \frac{\eta * v}{d}$$

Dabei steht F für den Fließwiderstand in Newton (N), $\eta$ für die Viskosität in Pascal-Sekunden (Pa * s), v für die Fließgeschwindigkeit in Meter pro Sekunde (m/s) und d für den Durchmesser des Fließkanals in Meter (m).

Entsprechend der vorstehend beschriebenen Vorgehensweise wurden im Labormaßstab weitere Rezepturen der erfindungsgemäßen Zubereitung hergestellt und getestet. Eine Zusammenstellung der bisher getesteten Varianten ist in Tabelle 2 enthalten, zusammen mit einer, aus den vorliegenden Beispielen extrapolierten allgemeinen erfindungsgemäßen Zusammensetzung, umfassend die Konzentrationsbereiche der Bestandteile Kollagen, Calciumhydroxid und Polyvinylpyrrolidon. Neben den konkret genannten Bestandteilen kann die erfindungsgemäße Zubereitung weitere Zusatzstoffe wie beispielsweise Stellmittel, Reaktionsverzögerer, Stabilisatoren, Puffersubstanzen und dergleichen enthalten. Damit werden die Eigenschaften der Zubereitung bzw. der Paste, welche aus der erfindungsgemäßen Zubereitung hergestellt wird, modifiziert (zum Beispiel Rieselfähigkeit, Haltbarkeit, Viskosität, pH-Wert). Die angegebenen oberen und unteren Grenzwerte der Konzentrationsbereiche sind daher so zu verstehen, dass sich die Gehalte jeder konkreten erfindungsgemäßen Mischung an den Bestandteilen Kollagen, Calciumhydroxid und Polyvinylpyrrolidon sowie destilliertem Wasser jeweils zu 100 Gewichtsprozent ergeben.

Tabelle 2: Übersicht über die erfindungsgemäßen Ausführungsbeispiele

| | | Kollagen | Calciumhydroxid | Polyvinylpyrrolidon PVP10 | destilliertes steriles Wasser |
|---|---|---|---|---|---|
| Beispiel 1 | Gramm | 3,37 | 4 | 2,25 | 2,0 |
| | Prozent | 27,6 | 32,7 | 18,4 | 17,2 |
| Beispiel 2 | Gramm | 3,75 | 4 | 2,25 | 2,2 |
| | Prozent | 29,8 | 31,7 | 17,9 | 17,5 |
| Beispiel 3 | Gramm | 3,75 | 4 | 2,35 | 2,2 |
| | Prozent | 29,1 | 31 | 18,2 | 17,1 |
| Mengenbereiche | Min. % | 27 | 30 | 17 | 16,5 |
| | Max. % | 31 | 34 | 19 | 18 |

Die Gewichtsprozente des Kollagens betragen 27-31% w/w, die Gewichtsprozente des Calciumhydroxids betragen 30-34% w/w, Gewichtsprozente des Polyvinylpyrrolidons betragen 17-19% w/w und die Gewichtsprozente des destillierten sterilen Wassers betragen 16,5-18% w/w.

Die Herstellung der erfindungsgemäßen Zubereitung und der aus der erfindungsgemäßen Zubereitung hergestellten erfindungsgemäßen Paste ist beliebig skalierbar, da die prozentualen Anteile der Substanzen konstant bleiben. Die Herstellung großer Chargen erfordert lediglich eine entsprechende Anpassung der Gerätschaften und eine kontinuierliche Überwachung der Mischprozesse, um die Homogenität und Konsistenz der erfindungsgemäßen Zubereitung und der aus der erfindungsgemäßen Zubereitung hergestellten erfindungsgemäßen Paste zu gewährleisten. Die Kontrolle der Viskosität und der Konsistenz der erfindungsgemäßen Zubereitung und der aus der erfindungsgemäßen Zubereitung hergestellten erfindungsgemäßen Paste kann durch regelmäßige Qualitätskontrollen sichergestellt werden.

Die erfindungsgemäße Zubereitung und die aus der erfindungsgemäßen Zubereitung hergestellten erfindungsgemäßen Paste finden Einsatz in der Chirurgie zur Verhinderung oder Behandlung von Gewebeinfektionen, insbesondere im Knochenbereich. Sie werden dabei prophylaktisch eingesetzt, indem sie vor der Implantation einer Endoprothese in das Operationsfeld hinzugefügt werden. Sie werden weiterhin therapeutisch im Frühstadium einer Infektion (innerhalb der ersten drei Wochen) eingesetzt, indem die erfindungsgemäße Zubereitung oder die aus der erfindungsgemäßen Zubereitung hergestellten erfindungsgemäßen Paste um den betreffenden Infektionsbereich herum aufgetragen werden, um das Implantat zu erhalten und seinen Verlust zu verhindern. Die erfindungsgemäße Zubereitung und die aus der erfindungsgemäßen Zubereitung hergestellten erfindungsgemäßen Paste werden auch eingesetzt, um nach der Entfernung von Endoprothesen die Periimplantitis im Operationsfeld zu behandeln. Ein weiterer möglicher Einsatz neben der Prophylaxe und Therapie von Periimplantitiden ist die prophylaktische Anwendung bei offenen Frakturen mit hohem Infektionsrisiko, als Erste-Hilfe-Maßnahme durch Sanitäter, z. B. bei Schusswunden oder bei Verkehrsunfällen. Die Anwendung ist vielfältig und umfasst alle medizinischen Behandlungen im Zusammenhang mit Periimplantitiden in Knochen oder Verletzungen mit freiliegenden Knochenbereichen (offene Frakturen).

**Ausführungsbeispiele**

**Hemmende Wirkung der einzelnen Komponenten der erfindungsgemäßen Zubereitung und der aus der erfindungsgemäßen Zubereitung hergestellten erfindungsgemäßen Paste auf E. coli-Bakterien**

**Versuch 1: Verhinderung des Bakterienwachstums**

Um zu überprüfen, ob die aus der erfindungsgemäßen Zubereitung hergestellte erfindungsgemäße Paste das Wachstum von E. coli-Bakterien verhindert, wurde der folgende Versuch durchgeführt. Eine Petrischale mit Agar-Agar-Nährboden wurde aus dem Kühlschrank entnommen und ein Abstrich der zuvor gezüchteten E. coli-Bakterien mit 1 ml destilliertem Wasser verdünnt. Dieser Abstrich wurde mit einem Drigalskispatel auf dem Nährboden verteilt. Die Petrischale wurde verschlossen und die Bakterien für bis zu 15 Minuten trocknen gelassen.

Um die Einzelwirkungen der einzelnen Komponenten der erfindungsgemäßen Zubereitung mit der Wirkung der der aus der erfindungsgemäßen Zubereitung hergestellten erfindungsgemäßen Paste zu vergleichen, wurden die einzelnen Komponenten mit Wasser zu Vergleichspasten (Calciumhydroxid-Vergleichspaste, Polyvinylpyrrolidon-Vergleichspaste, Kollagen-Vergleichspaste) angemischt.

Mit einer Schablone wurden mit einer sterilen Pipetten-Spitze Probelöcher in den Nährboden gestochen, um die Vergleichspasten und die aus der erfindungsgemäßen Zubereitung hergestellten erfindungsgemäßen Paste in fließfähiger Form und im ausgehärteten Zustand gleichmäßig zu verteilen (siehe Tabelle 3).

Tabelle 3: Komponenten der aus der erfindungsgemäßen Zubereitung hergestellten erfindungsgemäßen Paste einzeln und in erfindungsgemäßer Kombination sowie destilliertes steriles Wasser als Vergleichssubstanz (Negativkontrolle) für die präparierten Löcher im Agar-Agar-Nährboden

| Loch des Nährbodens | Komponenten der aus der erfindungsgemäßen Zubereitung hergestellten erfindungsgemäßen Paste einzeln und in erfindungsgemäßer Kombination | Menge |
|---|---|---|
| 1 | 4 g Calciumhydroxid in 1,0 ml destilliertem sterilem Wasser gelöst/suspendiert | 150 μl |
| 2 | 2,25 g Polyvinylpyrrolidon in 0,3 ml destilliertem sterilem Wasser gelöst/suspendiert | 150 μl |
| 3 | 3,37 g Kollagen in 0,7 ml destilliertem sterilem Wasser gelöst/suspendiert | 150 μl |
| 4 | Ausgehärtete Paste (37,5% Kollagen, 40% Calciumhydroxid, 22,5% Polyvinylpyrrolidon) in 2 ml destilliertem sterilem Wasser gelöst/suspendiert, anschließend 24 Stunden getrocknet gelassen. | 150 μl, nach Aushärtung entspricht 0,1 g |
| 5 | Fließfähige Paste (3,75 g Kollagen, 4 g Calciumhydroxid, 2,25 g Polyvinylpyrrolidon) in 2 ml destilliertem sterilem Wasser gelöst/suspendiert. | 150 μl |
| 6 | destilliertes steriles Wasser | 150 μl |

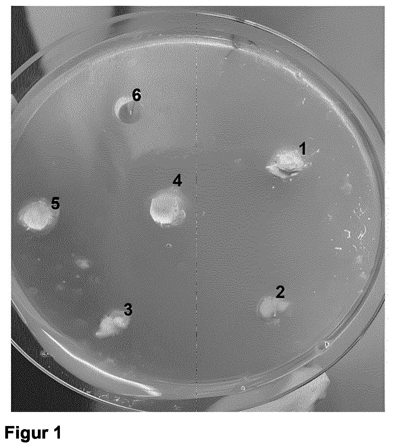

Die Löcher wurden wie folgt befüllt: In das erste Loch wurde mit Hilfe einer Pipette 150 μl Calciumhydroxid-Vergleichspaste pipettiert. Das zweite Loch wurde mit 150 μl Polyvinylpyrrolidon-Vergleichspaste befüllt und in das dritte Loch wurde 150 μl Kollagen-Vergleichspaste hinzugefügt. In das vierte Loch wurde 0,1 g der ausgehärteten der aus der erfindungsgemäßen Zubereitung hergestellten erfindungsgemäßen Paste gefüllt, was 150 μl der noch fließfähigen der aus der erfindungsgemäßen Zubereitung hergestellten erfindungsgemäßen Paste vor dem Aushärten entspricht. In das fünfte Loch des Agar-Agar-Nährbodens wurde 150 μl der fließfähigen der aus der erfindungsgemäßen Zubereitung hergestellten erfindungsgemäßen Paste gefüllt. In das sechste Loch wurden zur Kontrolle 150 μl destilliertes steriles Wasser pipettiert. Die Petrischale wurde verschlossen und für 8 Stunden bei optimaler Wachstumstemperatur für E. coli-Bakterien in den Inkubator gestellt. Nach dieser Inkubationszeit wurden die Ergebnisse dokumentiert, um festzustellen, ob das Wachstum der Bakterien verhindert wurde (siehe Figur 1).

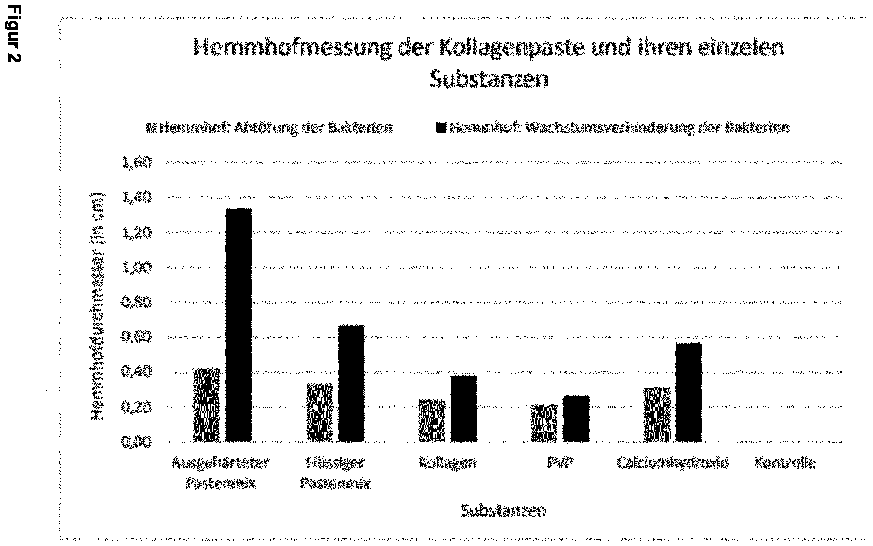

Figur 2 zeigt, dass beim Versuch 1 die radialen Ausdehnungen der Hemmhöfe bei der ausgehärteten der aus der erfindungsgemäßen Zubereitung hergestellten erfindungsgemäßen Paste einen Mittelwert von über 1,33 cm erreichen. Bei der fließfähigen der aus der erfindungsgemäßen Zubereitung hergestellten erfindungsgemäßen Paste lag der Mittelwert der radialen Ausdehnung der Hemmhöfe bei 0,66 cm. Die Kollagen-Vergleichspaste wies einen Mittelwert des Hemmhofdurchmessers von ca. 0,38 cm auf, während die Polyvinylpyrrolidon-Vergleichspaste den geringsten Wert mit einem Durchschnitt von nur 0,26 cm zeigte.

**Versuch 2: Abtötung der Bakterien**

Um zu überprüfen, ob die aus der erfindungsgemäßen Zubereitung hergestellte erfindungsgemäße Paste bereits vorhandene E. coli-Bakterien abtötet, wurde der zweite Versuch durchgeführt. Ein ausgehärteter Agar-Agar-Nährboden wurde aus dem Kühlschrank genommen. Ein Abstrich der gezüchteten E. coli-Bakterien wurde mit 1 ml destilliertem Wasser verdünnt und auf dem Nährboden verteilt. Die Petrischale wurde verschlossen und für 8 Stunden bei 37°C inkubiert, um das Bakterienwachstum zu fördern.

Nach dieser Inkubationszeit wurden mit einer sterilen Pipetten-Spitze Löcher in den Nährboden gestochen, wobei die gleiche Schablone wie in Versuch 1 verwendet wurde. Die Löcher wurden mit den gleichen Testsubstanzen wie im ersten Versuch befüllt. Anschließend wurde die Petrischale erneut verschlossen und für 24 Stunden in den Inkubator gestellt. Nach dieser zweiten Inkubationszeit wurde die Petrischale entnommen und die Ergebnisse dokumentiert, um festzustellen, ob die Bakterien abgetötet wurden.

Beim Versuch 2 sind die radialen Ausdehnungen der Hemmhöfe bei der ausgehärteten aus der erfindungsgemäßen Zubereitung hergestellten erfindungsgemäßen Paste kleiner und weisen einen Mittelwert von ca. 0,42 cm auf. Die fließfähige aus der erfindungsgemäßen Zubereitung hergestellte erfindungsgemäße Paste zeigte Hemmhofdurchmesser von durchschnittlich 0,33 cm. Das Kollagen-Vergleichspaste hatte im zweiten Versuch einen Mittelwert des Hemmhofdurchmessers von ca. 0,24 cm, während das Polyvinylpyrrolidon-Vergleichspaste im Durchschnitt nur einen Hemmhofdurchmesser von 0,21 cm aufwies.

**Versuch 3: Einfluss der aus der erfindungsgemäßen Zubereitung hergestellten erfindungsgemäßen Paste auf Zellen von Säugetieren**

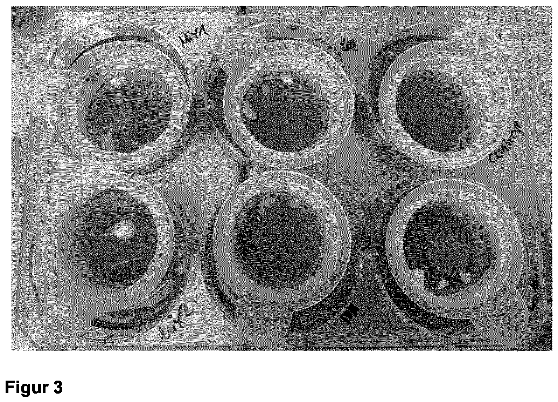

Im Versuch 3 wurde der Einfluss der aus der erfindungsgemäßen Zubereitung hergestellte erfindungsgemäße Paste auf mesenchymale Stammzellen von Ratten untersucht. Damit wird die Verträglichkeit der erfindungsgemäßen Zubereitung und der aus der erfindungsgemäßen Zubereitung hergestellten erfindungsgemäßen Paste auf lebende Zellen belegt. Zu Beginn wurden die Zellen mit einem CASY®-Zellzähler gezählt. Die Zellen wurden dann in eine 6-Well-Kulturplatte überführt, und die Testsubstanzen wurden in ein Sieb über die Zellen, aber in Kontakt mit dem Medium aufgetragen. Eine Kontrollprobe mit leerem Zellsieb wurde ebenfalls angelegt (Figur 3). Die Zellkulturplatte wurde verschlossen und bei 37° C inkubiert. Nach 24 Stunden wurden die Zellen überprüft.

Die anfängliche Zellzahl betrug etwa 101.000, mit 88.600 lebenden und 12.400 toten Zellen. Nach 24 Stunden wiesen die Kontrollproben durchschnittlich 468.313 Zellen/mL auf, davon 411.275 lebend und 57.038 tot. Die fließfähige aus der erfindungsgemäßen Zubereitung hergestellte erfindungsgemäße Paste führte zu einem signifikanten Zellwachstum auf 1,3 Millionen Zellen/mL, davon 937.500 lebende Zellen. Die ausgehärtete aus der erfindungsgemäßen Zubereitung hergestellte erfindungsgemäße Paste zeigte ein geringeres Wachstum mit 754.800 Zellen/mL, davon 665.500 lebend. Diese Ergebnisse zeigen, dass die fließfähige aus der erfindungsgemäßen Zubereitung hergestellte erfindungsgemäße Paste das Wachstum von Stammzellen fördert, woraus sich die knochenregenerierende Wirkung der aus der erfindungsgemäßen Zubereitung hergestellte erfindungsgemäße Paste ableiten lässt.

Die Calciumhydroxid-Vergleichspaste ergab 482.000 Zellen, davon 442.000 lebend. Die Polyvinylpyrrolidon-Vergleichspaste und die Kollagen-Vergleichspaste zeigten ein geringeres Wachstum als die Kontrolle. Die Polyvinylpyrrolidon-Vergleichspaste hatte 365.000 Zellen (329.000 lebend), und die Kollagen-Vergleichspaste hatte 252.000 Zellen (218.000 lebend). Die Berechnung des prozentualen Wachstums erfolgt nach folgender Formel:

Prozentuales Wachstum = (Anzahl der lebenden Zellen nach 24 Stunden / Gesamtanzahl der Zellen zum Zeitpunkt 0) $\times$ 100

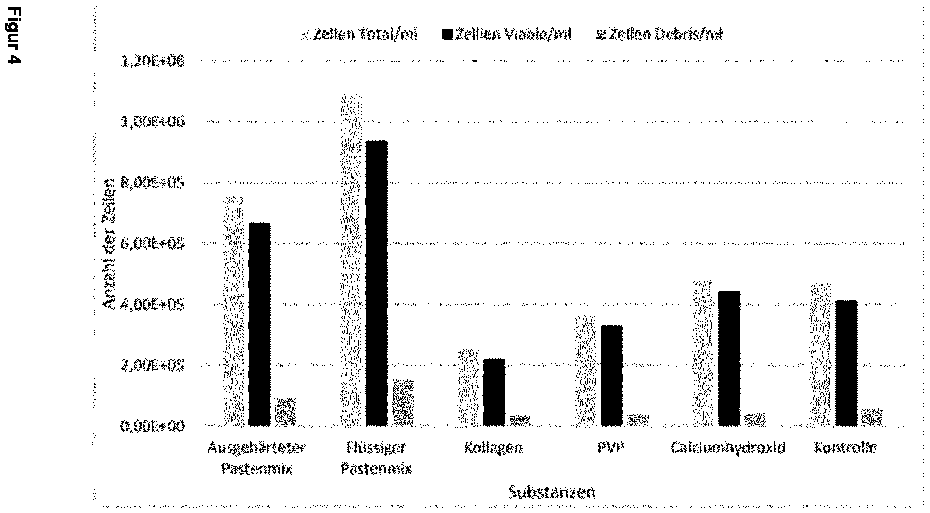

Die aus der erfindungsgemäßen Zubereitung hergestellte erfindungsgemäße Paste zeigte das höchste prozentuale Wachstum der lebenden Zellen. Diese Daten sind in Figur 4 als Balkendiagramm dargestellt. Insgesamt zeigte die aus der erfindungsgemäßen Zubereitung hergestellte erfindungsgemäße Paste die größte Verbesserung des Zellwachstums im Vergleich zu den anderen Komponenten und der Kontrolle.

**Versuch 4: Viskositätsuntersuchung der aus der erfindungsgemäßen Zubereitung hergestellten erfindungsgemäßen Paste**

Um zu untersuchen, wie sich die Fließfähigkeit der aus der erfindungsgemäßen Zubereitung hergestellten erfindungsgemäßen Paste in Abhängigkeit zur Lagerungsdauer verändert, wurde die Viskosität der aus der erfindungsgemäßen Zubereitung hergestellte erfindungsgemäße Paste über den Lauf der Zeit hinweg überprüft. Dazu wurde die aus der erfindungsgemäßen Zubereitung hergestellte erfindungsgemäße Paste zu verschiedenen Zeitpunkten angemischt:

Nach einem Tag (D1), sowie nach einer Woche (1W), zwei Wochen (2W) und drei Wochen (3W). Diese fließfähigen aus der erfindungsgemäßen Zubereitung hergestellten erfindungsgemäßen Pasten wurden jeweils drei, zwei und eine Woche sowie einen Tag vor der Viskositätsprüfung hergestellt. Damit konnten die Untersuchungen zur Veränderung der Fließfähigkeit für die unterschiedlich alten fließfähigen aus der erfindungsgemäßen Zubereitung hergestellten erfindungsgemäßen Pasten am selben Tag durchgeführt werden. Es wurden jeweils 2 ml der fließfähigen aus der erfindungsgemäßen Zubereitung hergestellten erfindungsgemäßen Pasten in 5 ml Einmalspritzen gefüllt, luftdicht verschlossen (z.B. mit Parafilm®), sowie dunkel und bei ca. 20° C (= Raumtemperatur) gelagert, damit sie nicht zuvor aushärten. Für die Viskositätsuntersuchung wurden pro Lagerungsdauer jeweils acht Proben gemessen.

Die Viskosität der fließfähigen aus der erfindungsgemäßen Zubereitung hergestellten erfindungsgemäßen Pasten in Abhängigkeit von der Lagerungsdauer wurde mittels einer Materialprüfmaschine (z.B. der Firma ZwickRoell) sowie einer Prüfsoftware (z.B. testXpert® III) untersucht. Die Untersuchung wurde an der Materialprüfmaschine ausgeführt. Zu Beginn wurden alle Spritzen von dem Parafilm® gelöst und jeweils in die Prüfmaschine eingespannt. Die Testgeschwindigkeit betrug 5 mm/min. Die Einmalspritze wurde in der Prüfmaschine senkrecht und überkopf in einer Halterung fixiert, sodass der Stempel der Maschine mit einer axialen Kraft auf den Kolben der Einmalspritze aufkommt und die fließfähige aus der erfindungsgemäßen Zubereitung hergestellte erfindungsgemäße Paste aus der Einmalspritze herausdrückt. Die Ergebnisse, mit welcher Kraft (N) der Spritzenkolben heruntergedrückt wird, um die fließfähige aus der erfindungsgemäßen Zubereitung hergestellte erfindungsgemäße Paste herauszudrücken, wurden dokumentiert.

Anhand der Ergebnisse wird gezeigt, wie sich die Viskosität der aus der erfindungsgemäßen Zubereitung hergestellte erfindungsgemäße Paste in Abhängigkeit von der Lagerungsdauer verändert.

Die Kraft (N), die benötigt wurde, um die fließfähige aus der erfindungsgemäßen Zubereitung hergestellte erfindungsgemäße Paste zu verdrängen, stieg erheblich von D1 bis 3W. Die Gruppe D1 zeigte die niedrigsten Kraftwerte, was darauf hindeutet, dass die fließfähige aus der erfindungsgemäßen Zubereitung hergestellte erfindungsgemäße Paste nach einem Tag am leichtesten zu verdrängen war. Mit zunehmender Aushärtungszeit stiegen die Kraftwerte an, wobei die Gruppe 3W die höchsten Werte aufwies. Dies deutet darauf hin, dass die Paste im Laufe der Zeit deutlich widerstandsfähiger gegen Verdrängung wird, wahrscheinlich aufgrund einer erhöhten Kohäsion und strukturellen Integrität während des Aushärtungsprozesses.

Die Verschiebungswerte (m) variierten ebenfalls deutlich über die Zeit. Nach einem Tag (D1) zeigte die fließfähige aus der erfindungsgemäßen Zubereitung hergestellte erfindungsgemäße Paste die höchste Verschiebung, was auf eine höhere Verformbarkeit hinweist. Mit zunehmender Aushärtungszeit auf eine Woche (1W), zwei Wochen (2W) und drei Wochen (3W) nahmen die Verschiebungswerte ab, wobei die geringste Verschiebung in der Gruppe 3W beobachtet wurde. Diese Abnahme der Verschiebung im Laufe der Zeit korreliert mit dem Anstieg der Kraft, was weiter unterstützt, dass die fließfähige aus der erfindungsgemäßen Zubereitung hergestellte erfindungsgemäße Paste steifer und weniger verformbar wird, je länger sie aushärtet.

Die Fließrate ($m^3$/s) der fließfähigen aus der erfindungsgemäßen Zubereitung hergestellten erfindungsgemäßen Paste zeigte einen abnehmenden Trend von D1 bis 3W. Die Gruppe D1 hatte die höchste Fließrate, was darauf hindeutet, dass die fließfähige aus der erfindungsgemäßen Zubereitung hergestellte erfindungsgemäße Paste nach einem Tag am flüssigsten war. Mit fortschreitender Aushärtung über Wochen nahm die Fließrate ab, wobei die Gruppe 3W die niedrigste Fließrate aufwies. Dies deutet auf eine signifikante Erhöhung der Viskosität und eine Verringerung der Fließfähigkeit hin, da die fließfähige aus der erfindungsgemäßen Zubereitung hergestellte erfindungsgemäße Paste aushärtet.

Die Scherrate ($s^{-1}$) und die Scherspannung (Pa) stiegen beide mit der Aushärtungszeit. Die Gruppe D1 hatte die niedrigste Scherrate und Scherspannung, während die Gruppe 3W die höchsten Werte aufwies. Der Anstieg der Scherrate und Scherspannung im Laufe der Zeit zeigt, dass der Widerstand der fließfähigen aus der erfindungsgemäßen Zubereitung hergestellten erfindungsgemäßen Paste gegen Scherkräfte wächst, während sie aushärtet, was auf eine erhöhte interne Reibung und strukturelle Stabilität hinweist. Diese Änderungen stimmen mit den beobachteten Kraftanstiegen und den Abnahmen der Verschiebung und Fließrate überein. Die Viskositätswerte (Pa s) nahmen von D1 bis 3W erheblich zu, wobei die Gruppe 1D die niedrigste Viskosität und die Gruppe 3W die höchste aufwies. Diese Zunahme der Viskosität im Laufe der Zeit deutet darauf hin, dass die fließfähige aus der erfindungsgemäßen Zubereitung hergestellte erfindungsgemäße Paste dicker und widerstandsfähiger gegen Fließen wird, während sie aushärtet. Die höhere Viskosität in den späteren Wochen stimmt mit den niedrigeren Fließraten und den höheren Kräften überein, die für die Verdrängung erforderlich sind, was den Trend einer zunehmenden Materialsteifigkeit und einer verringerten Fließfähigkeit unterstützt.

Die Ergebnisse zeigen signifikante Unterschiede in den Fließfähigkeitseigenschaften der fließfähigen aus der erfindungsgemäßen Zubereitung hergestellten erfindungsgemäßen Paste, wenn sie nach den verschiedenen Zeiträumen getestet wird (ein Tag (D1), eine Woche (1W), zwei Wochen (2W) und drei Wochen (3W)). Jede Variable zeigte deutliche Trends und Unterschiede über diese Zeitpunkte hinweg, die wertvolle Einblicke in die zeitlichen Veränderungen der Materialeigenschaften liefern.

Die Kraft, die bei konstanter Geschwindigkeit erforderlich ist, um die fließfähige aus der erfindungsgemäßen Zubereitung hergestellte erfindungsgemäße Paste zu verdrängen, nahm von D1 bis 3W erheblich zu. Dies deutet darauf hin, dass die fließfähige aus der erfindungsgemäßen Zubereitung hergestellte erfindungsgemäße Paste im Laufe der Zeit signifikant widerstandsfähiger gegen Verdrängung wird, wahrscheinlich aufgrund einer erhöhten Kohäsion und strukturellen Integrität während der Lagerung.

Die Verschiebungswerte variierten ebenfalls deutlich über die Zeit. Nach einem Tag (D1) zeigte die fließfähige aus der erfindungsgemäßen Zubereitung hergestellte erfindungsgemäße Paste die höchste Verschiebung, was auf eine höhere Verformbarkeit hinweist. Mit zunehmender Lagerungszeit auf eine Woche (1W), zwei Wochen (2W) und drei Wochen (3W) nahmen die Verschiebungswerte ab, wobei die geringste Verschiebung in der Gruppe 3W beobachtet wurde. Diese Abnahme der Verschiebung im Laufe der Zeit korreliert mit dem Anstieg der Kraft, was weiter unterstützt, dass die fließfähige aus der erfindungsgemäßen Zubereitung hergestellte erfindungsgemäße Paste steifer und weniger verformbar wird, je länger sie lagert.

Die Fließrate der fließfähigen aus der erfindungsgemäßen Zubereitung hergestellten erfindungsgemäßen Paste zeigte einen abnehmenden Trend von D1 bis 3W. Dies deutet auf eine signifikante Erhöhung der Viskosität und eine Verringerung der Fließfähigkeit hin, da die fließfähige aus der erfindungsgemäßen Zubereitung hergestellte erfindungsgemäße Paste während der Lagerung aushärtet und es schwieriger wird, dass das Material fließt.

Die Scherrate und die Scherspannung stiegen beide mit der Lagerungszeit. Der Anstieg der Scherrate und Scherspannung im Laufe der Zeit zeigt, dass der Widerstand der fließfähigen aus der erfindungsgemäßen Zubereitung hergestellten erfindungsgemäßen Paste gegen Scherkräfte wächst, während sie lagert, was auf eine erhöhte interne Reibung und strukturelle Stabilität hinweist. Diese Änderungen stimmen mit den beobachteten Kraftanstiegen und den Abnahmen der Verschiebung und Fließrate überein.

Die Viskositätswerte nahmen von D1 bis 3W erheblich zu, was darauf hinweist, dass die fließfähige aus der erfindungsgemäßen Zubereitung hergestellte erfindungsgemäße Paste dicker und widerstandsfähiger gegen Fließen wird, während sie lagert. Die höhere Viskosität in den späteren Wochen stimmt mit den niedrigeren Fließraten und den höheren Kräften überein, die für die Verdrängung erforderlich sind, was den Trend einer zunehmenden Materialsteifigkeit und einer verringerten Fließfähigkeit unterstützt.

Die Testungen zeigen, dass sich die Fließfähigkeitseigenschaften der fließfähigen aus der erfindungsgemäßen Zubereitung hergestellten erfindungsgemäßen Paste im Laufe der Zeit erheblich ändern. Nach einem Tag ist die fließfähige aus der erfindungsgemäßen Zubereitung hergestellte erfindungsgemäße Paste relativ leicht zu verdrängen, hoch verformbar und weist hohe Fließraten sowie niedrige Viskosität, Scherrate und Scherspannung auf. Mit fortschreitender Lagerung über eine Woche, zwei Wochen und drei Wochen wird die fließfähige aus der erfindungsgemäßen Zubereitung hergestellte erfindungsgemäße Paste signifikant widerstandsfähiger gegen Verdrängung, weniger verformbar, mit niedrigeren Fließraten und höheren Viskositäten, Scherraten und Scherspannungen. Diese Erkenntnisse zeigen, dass die fließfähige aus der erfindungsgemäßen Zubereitung hergestellte erfindungsgemäße Paste während des Lagerungsprozesses wesentliche Änderungen ihrer physikalischen Eigenschaften erfährt.

Die erfindungsgemäße Zubereitung und die aus der erfindungsgemäßen Zubereitung hergestellte erfindungsgemäße Paste können prophylaktisch zur Verhinderung von Gewebeinfektionen angewendet werden, insbesondere bei Knochen. Das gilt sowohl für die Anwendung zur Verhinderung einer Periimplantitis, bei der Implantation einer Endoprothese als auch bei anderen Verletzungen von Knochen oder anderen Geweben wie beispielsweise offene Frakturen, Schusswunden oder andere Verletzungen.

Die erfindungsgemäße Zubereitung kann therapeutisch zur Behandlung einer Gewebeinfektionen angewendet werden insbesondere bei Knochen. Das gilt sowohl für die Anwendung zur Behandlung einer Periimplantitis nach einer Implantation einer Endoprothese als auch bei Infektionen von Knochen oder anderen Geweben nach Verletzungen wie beispielsweise offenen Frakturen, Schusswunden oder anderen Verletzungen.

**Abbildungslegende**

**Figur 1** zeigt die Agar-Petrischale mit Bakterien und Löchern mit den Komponenten der erfindungsgemäßen Zubereitung bzw. der aus der erfindungsgemäßen Zubereitung hergestellten erfindungsgemäßen Paste: Loch 1 = Calciumhydroxid-Vergleichspaste, Loch 2 = Polyvinylpyrrolidon-Vergleichspaste, Loch 3 = Kollagen-Vergleichspaste, Loch 4 = ausgehärtete aus der erfindungsgemäßen Zubereitung hergestellte erfindungsgemäße Paste, Loch 5 = fließfähige aus der erfindungsgemä-βen Zubereitung hergestellte erfindungsgemäße Paste, Loch 6 = destilliertes steriles Wasser.

**Figur 2** zeigt die Auswirkung der aus der erfindungsgemäßen Zubereitung hergestellten erfindungsgemäßen Paste und der Vergleichspasten auf E. coli-Bakterien. Dargestellt ist die radiale Ausdehnung der Hemmhöfe (Hemmhofdurchmesser) in cm.

**Figur 3** zeigt die Auswirkung der aus der erfindungsgemäßen Zubereitung hergestellten erfindungsgemäßen Paste und der Vergleichspasten auf mesenchymale Stammzellen von Ratten.

**Figur 4** zeigt die Anzahl der Zellen nach Inkubation mit den Vergleichspasten und der aus der erfindungsgemäßen Zubereitung hergestellten erfindungsgemäßen Paste (fließfähig und ausgehärtet).

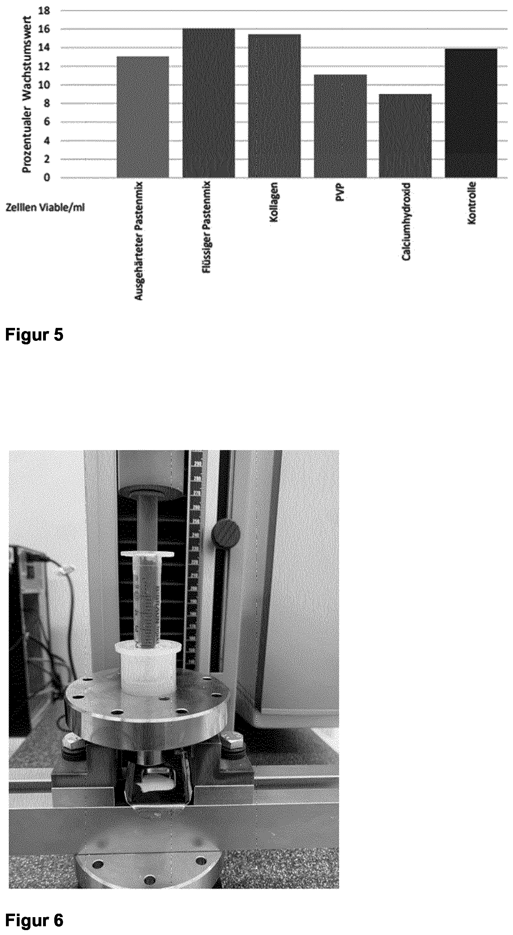

**Figur 5** zeigt das prozentuale Wachstum der Gesamtanzahl der Zellen zu den lebenden Zellen nach Inkubation mit den Vergleichspasten und der aus der erfindungsgemäßen Zubereitung hergestellten erfindungsgemäßen Paste (fließfähig und ausgehärtet).

**Figur 6** zeigt die Einmalspritze mit der fließfähigen der aus der erfindungsgemäßen Zubereitung hergestellten erfindungsgemäßen Paste, eingesetzt in eine Materialprüfmaschine.

## Patentansprüche

1. Antimikrobielle Zubereitung umfassend mindestens Calciumhydroxid, Polyvinylpyrrolidon (PVP) und Kollagen.

2. Antimikrobielle Zubereitung gemäß Anspruch 1, **dadurch gekennzeichnet, dass** sie 30-40% w/w Calciumhydroxid, 17-22,5% w/w Polyvinylpyrrolidon (PVP) und 27-37,5% w/w Kollagen umfasst.

3. Antimikrobielle Zubereitung gemäß Anspruch 1 und 2, **dadurch gekennzeichnet, dass** sie 40,0% w/w Calciumhydroxid, 22,5% w/w Polyvinylpyrrolidon (PVP) und 37,5% w/w Kollagen umfasst.

4. Antimikrobielle Zubereitung gemäß einem der Ansprüche 1 bis 3, **dadurch gekennzeichnet, dass** das verwendete Polyvinylpyrrolidon (PVP) ein Polyvinylpyrrolidon mit einem Molekulargewicht von 10.000 Dalton (PVP10) ist.

5. Antimikrobielle Zubereitung gemäß Anspruch 4, **dadurch gekennzeichnet, dass** zum PVP10 wenigstens ein weiteres Polyvinylpyrrolidon, ausgewählt aus der Gruppe PVP40 (mit einem Molekulargewicht von 40.000 Dalton), PVP K90 (mit einem Molekulargewicht von 90.000 Dalton) oder PVP K30 (mit einem Molekulargewicht von 30.000 Dalton), verwendet wird.

6. Verfahren zur Herstellung einer Paste umfassend die Schritte

    i. Bereitstellen einer antimikrobiellen Zubereitung gemäß einem der Ansprüche 1 bis 5
    ii. Bereitstellen von Wasser
    iii. Vermischen der Zubereitung aus Schritt i. mit dem Wasser aus Schritt ii., so dass eine fließfähige Paste entsteht.

7. Verfahren zur Herstellung einer Paste gemäß Anspruch 6, **dadurch gekennzeichnet, dass** das Wasser aus Schritt ii. destilliertes und/oder steriles Wasser ist.

8. Verfahren zur Herstellung einer Paste gemäß der Ansprüche 6 und 7, wobei die Gewichtsprozente des Kollagens 27-31% w/w betragen, die Gewichtsprozente des Calciumhydroxids 30-34% w/w betragen, die Gewichtsprozente des Polyvinylpyrrolidons (PVP) 17-19% w/w betragen und die Gewichtsprozente des Wassers 16,5-18% w/w betragen.

9. Verfahren zur Herstellung einer Paste gemäß Anspruch 6, **dadurch gekennzeichnet, dass** zusätzlich zu den Schritten i. bis iii. in einem weiteren Schritt iv. Glycerin und/oder Polyethylenglykol (PEG) zugefügt wird, um die Fließeigenschaften der Paste anzupassen.

10. Verfahren zur Herstellung einer Paste gemäß der Ansprüche 6 bis 9, **dadurch gekennzeichnet, dass** zusätzlich zu den Schritten i. bis iv. in einem weiteren Schritt v. Zusatzstoffe wie beispielsweise Stellmittel, Reaktionsverzögerer, Puffersubstanzen und/oder Stabilisatoren zugefügt werden, um die Eigenschaften der Paste zu modifizieren.

11. Verwendung der antimikrobiellen Zubereitung nach einem der Ansprüche 1 bis 5 und/oder der Paste, hergestellt gemäß einem der Ansprüche 6 bis 10, für die Herstellung einer pharmazeutischen Zubereitung.

12. Verwendung der pharmazeutischen Zubereitung aus Anspruch 11 als Medikament zur Prophylaxe von Gewebeinfektionen.

13. Verwendung der pharmazeutischen Zubereitung aus Anspruch 11 als Medikament zur Therapie von Gewebeinfektionen.

14. Verwendung der pharmazeutischen Zubereitung aus Anspruch 11 als Medikament gegen Gewebeinfektionen im Knochenbereich.

**Figur 1**

**Figur 2**

**Figur 3**

**Figur 4**

EP 4 717 281 A1

Figur 5

Figur 6

EP 4 717 281 A1

# EUROPÄISCHER RECHERCHENBERICHT

Nummer der Anmeldung

EP 24 20 3666

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2002/198283 A1 (IMAI YOHJI [JP] ET AL) 26. Dezember 2002 (2002-12-26) * Anspruch 1 * * Beispiel 1 * ----- | 1-14 | INV. A61L27/02 A61L27/26 A61L27/50 A61L27/58 A61K6/58 A61K33/08 |
| A | CN 108 785 105 A (SINO DENTEX CO LTD) 13. November 2018 (2018-11-13) * Beispiel 1 * ----- | 1-14 | |
| A | WO 2023/201879 A1 (KUNMING QINGCHENG MEDICAL TECH LTD [CN]) 26. Oktober 2023 (2023-10-26) * Anspruch 8 * ----- | 1-14 | |
| A | PISSIOTIS ELEFTHERIA ET AL: "<mark>Biological evaluation of collagen gels containing calcium hydroxide and hydroxyapatite</mark>", JOURNAL OF ENDODONTICS, Bd. 16, Nr. 10, 1. Oktober 1990 (1990-10-01), Seiten 468-473, XP093257851, AMSTERDAM, NL ISSN: 0099-2399, DOI: 10.1016/S0099-2399(07)80175-0 * Zusammenfassung * ----- | 1-14 | |
| X | EP 1 880 713 A1 (METACURA FZE [AE]) 23. Januar 2008 (2008-01-23) * Ansprüche 1,10,11,13,17 * * Absatz [0007] * ----- | 1-5, 11-13 | |

RECHERCHIERTE SACHGEBIETE (IPC)

A61L
A61K

Der vorliegende Recherchenbericht wurde für alle Patentansprüche erstellt

| Recherchenort | Abschlußdatum der Recherche | Prüfer |
|---|---|---|
| Den Haag | 17. März 2025 | Zalfen, Alina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

21

**ANHANG ZUM EUROPÄISCHEN RECHERCHENBERICHT
ÜBER DIE EUROPÄISCHE PATENTANMELDUNG NR.**

EP 24 20 3666

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-03-2025

| Im Recherchenbericht angeführtes Patentdokument | Datum der Veröffentlichung | Mitglied(er) der Patentfamilie | Datum der Veröffentlichung |
|---|---|---|---|
| US 2002198283 A1 | 26-12-2002 | DE 10104557 A1<br>GB 2359817 A<br>US 2002198283 A1 | 09-08-2001<br>05-09-2001<br>26-12-2002 |
| CN 108785105 A | 13-11-2018 | KEINE | |
| WO 2023201879 A1 | 26-10-2023 | CN 114767548 A<br>WO 2023201879 A1 | 22-07-2022<br>26-10-2023 |
| EP 1880713 A1 | 23-01-2008 | DE 102006032887 A1<br>EP 1880713 A1 | 17-01-2008<br>23-01-2008 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3273948 B1
- EP 2683421 B1
- EP 1244434 B1